(19) Europäisches Patentamt
European Patent Office
Office européen des brevets

(11) **EP 3 871 611 B1**

(12) **EUROPEAN PATENT SPECIFICATION**

(45) Date of publication and mention
of the grant of the patent:
**22.11.2023 Bulletin 2023/47**

(21) Application number: **19877409.3**

(22) Date of filing: **21.10.2019**

(51) International Patent Classification (IPC):
*G16H 50/50* (2018.01)    *A61B 8/14* (2006.01)

(52) Cooperative Patent Classification (CPC):
**A61B 8/085; A61B 8/14; A61B 8/4483;
A61B 8/5223; G01S 7/52036; G01S 15/8922;
G06N 3/045; G06N 3/08; G16H 30/20; G16H 30/40;
G16H 50/20; G16H 50/50; G16H 50/70**

(86) International application number:
**PCT/JP2019/041335**

(87) International publication number:
**WO 2020/085312 (30.04.2020 Gazette 2020/18)**

(54) **ULTRASONIC DIAGNOSTIC SYSTEM**

ULTRASCHALLDIAGNOSESYSTEM

SYSTÈME DE DIAGNOSTIC ULTRASONORE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **22.10.2018 JP 2018198658**

(43) Date of publication of application:
**01.09.2021 Bulletin 2021/35**

(73) Proprietor: **Lily Medtech Inc.
Tokyo 113-0033 (JP)**

(72) Inventors:
• **TOMII, Naoki**
**Tokyo 113-0033 (JP)**
• **NAKAMURA, Hirofumi**
**Tokyo 113-0033 (JP)**

(74) Representative: **Hoffmann Eitle
Patent- und Rechtsanwälte PartmbB
Arabellastraße 30
81925 München (DE)**

(56) References cited:
**US-A1- 2018 206 827**

• **MICHA FEIGIN ET AL: "A Deep Learning
Framework for Single-Sided Sound Speed
Inversion in Medical Ultrasound", ARXIV.ORG,
CORNELL UNIVERSITY LIBRARY, 201 OLIN
LIBRARY CORNELL UNIVERSITY ITHACA, NY
14853, 30 September 2018 (2018-09-30),
XP081451311, DOI: 10.1109/TBME.2019.2931195**
• **VEDULA, SANKETH et al.: "TOWARDS
CT-QUALITY ULTRASOUND IMAGING USING
DEEP LEARNING", arXiv , 1710.06304v1, 17
October 2017 (2017-10-17), XP080825022,**
• **KUTTER, OLIVER et al.: "Visualization and GPU-
accelerated simulation of medical ultrasound
from CT images", Computer Methods and
Programs in Biomedicine, vol. 94, 2009, pages
250-266, XP026033561, DOI:
10.1016/j.cmpb.2008.12.011**
• **CHENG, H. D. et al.: "Automated breast cancer
detection and classification using ultrasound
images= A survey", Pattern Recognition, vol. 43,
2010, pages 299-317, XP026498170, DOI:
10.1016/j.patcog.2009.05.012**

**Description**

Technical Field

The present invention relates to an ultrasound diagnostic system in which ultrasound irradiation is performed and a tomographic image of a test object is generated.

Background Art

A noninvasive diagnostic system using ultrasound is widely used in the medical field as a technology for making a diagnosis based on information regarding the inside of a test object since there is no need to perform surgery in which a direct incision is made to carry out an observation in a living body.

In ultrasound computed tomography (CT), which is a technique for making a diagnosis using ultrasound, a test object is irradiated with ultrasound and a tomographic image of the test object is generated using reflected ultrasound or transmitted ultrasound. A recent study shows that ultrasound CT is useful in detecting breast cancer. In ultrasound CT, for example, a ring array transducer obtained by arranging, in a ring shape, many elements that emit and receive ultrasound is used to generate tomographic images.

For example, while switching an element that emits ultrasound in order, echo signals are received by all the elements and are stored as RF data (raw data). An image signal representing a tomographic image is then generated on the basis of the RF data.

Hitherto, when a tomographic image is reconstructed, approximation calculations such as the sound speed being treated as a constant have been performed. The amount of information of RF data is reduced through the approximation calculations, and thus it becomes difficult to form a clear tomographic image, resulting in an impediment to improving the accuracy with which a diagnosis is made.

MICHA FEIGIN ET AL: "A Deep Learning Framework for Single-Sided Sound Speed Inversion in Medical Ultrasound", ARXIV.ORG, CORNELL UNIVERSITY LIBRARY, 201 OLIN LIBRARY CORNELL UNIVERSITY ITHACA, NY 14853, 30 September 2018 (2018-09-30) relates to a single-sided sound speed inversion solution using a fully convolutional deep neural network. Simulations are used for training in order to allow the generation of limitless ground truth data.

VEDULA, SANKETH et al.: "TOWARDS CT-QUALITY ULTRASOUND IMAGING USING DEEP LEARNING", arXiv, 1710.06304v1, 17 October 2017 (2017-10-17) relates to CT-quality ultrasound imaging using deep learning. A convolutional neural network (CNN) is used to approximate a CT-quality image of a tissue given its corresponding ultrasound image. The CNN can reconstruct a CT-quality image from ultrasound IQ images, wherein the CNN is trained and tested on pairs of CT images and the corresponding simulated ultrasound images.

CHENG, H. D. et al.: "Automated breast cancer detection and classification using ultrasound images= A survey", Pattern Recognition, vol. 43, 2010, pages 299-317 relates to a CAD system which consists of four stages: preprocessing, segmentation, feature extraction and selection, and classification. The approaches used in these stages are summarized and their advantages and disadvantages are discussed. Furthermore, the performance evaluation of the CAD system is investigated.

PTL 1: International Publication No. 2017/051903

Summary of Invention

The present invention has been made in light of the existing circumstances described above, and an object of the present invention is to provide an ultrasound diagnostic system that generates a clear, accurate ultrasound image.

The present invention is defined in the appended independent claims. Further preferred embodiments are defined in the dependent claims.

Advantageous Effects of Invention

According to the present invention, a clear, accurate ultrasound image can be generated.

Brief Description of Drawings

[Fig. 1] Fig. 1 is a schematic diagram of the configuration of an ultrasound diagnostic system according to an embodiment of the present invention.

[Fig. 2] Fig. 2 is a section view taken along line A-A of Fig. 1.

[Fig. 3] Fig. 3 is a functional block diagram of a calculation device.

[Fig. 4] Fig. 4 is a schematic diagram of measurement data.

[Fig. 5] Fig. 5 is a schematic diagram of propagation of ultrasound.

[Fig. 6] Fig. 6 is a diagram illustrating partial RF data of a point of interest.

[Fig. 7] Fig. 7 is a schematic diagram of the structure of a learner.

[Fig. 8] Fig. 8 is a schematic diagram illustrating an image reconstruction method.

[Fig. 9] Fig. 9a illustrates a biological model, Fig. 9b illustrates a measurement image, and Fig. 9c is a diagram illustrating an image reconstructed from an output from the learner.

[Fig. 10] Fig. 10 is a diagram illustrating an example of emission and reception at a plurality of elements.

[Fig. 11] Fig. 11 is a diagram illustrating an example of partial RF data.

[Fig. 12] Figs. 12a and 12b are diagrams illustrating examples of propagation paths of ultrasound, and Fig. 12c is a diagram illustrating partial RF data.

[Fig. 13] Fig. 13 is a diagram illustrating an example in which a brightness image is converted into a density distribution.

[Fig. 14] Fig. 14a illustrates a brightness image, Fig. 14b illustrates a density distribution image, and Fig. 14c illustrates a reconstructed image.

Description of Embodiments

In the following, the present invention will be described in more detail with reference to the drawings. An ultrasound diagnostic system according to an embodiment of the present invention irradiates a test object such as a human body with ultrasound and generates a tomographic image (an ultrasound image) using received reflected-wave signals. A doctor makes a diagnosis by checking the generated tomographic image.

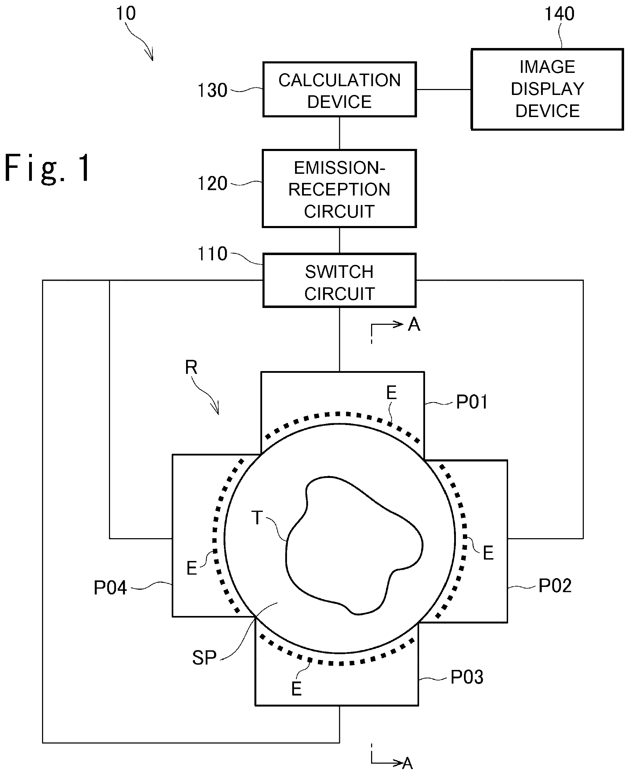

As illustrated in Fig. 1, an ultrasound diagnostic system 10 according to the present embodiment includes a ring array R, a switch circuit 110, an emission-reception circuit 120, a calculation device 130, and an image display device 140.

The ring array R is a ring-shaped transducer constituted by a combination of a plurality of transducers and having preferably a diameter of 80 to 500 mm and more preferably a diameter of 100 to 300 mm. The ring array R may have a variable diameter. In the present embodiment, as an example, a ring-shaped transducer obtained by combining four concave transducers P01 to P04 is used.

For example, in a case where the concave transducers P01 to P04 each have 512 rectangular piezoelectric elements E (hereinafter also simply referred to as "elements E"), the ring array R is constituted by 2048 elements E. The number of elements E provided at the concave transducers P01 to P04 is not limited to a specific number and is preferably between 1 and 1000.

Each element E has the function of converting an electrical signal into an ultrasonic signal and converting an ultrasonic signal into an electrical signal. The element E emits ultrasound to a test object T, receives reflected waves that are waves reflected by the test object T, and forms an electrical signal as measurement data.

In the present embodiment, each element E is described as an element having the function of both emitting and receiving ultrasound; however, the element E is not limited to this. For example, emission elements or reception elements may be used, which have either one of the function of emitting ultrasound and the function of receiving ultrasound, and a plurality of emission elements and a plurality of reception elements may be arranged in a ring shape. In addition, the ring array R may be constituted by the element (or elements) having the function of both emitting and receiving ultrasound, the emission element (or elements), and the reception element (or elements) in a mixed manner.

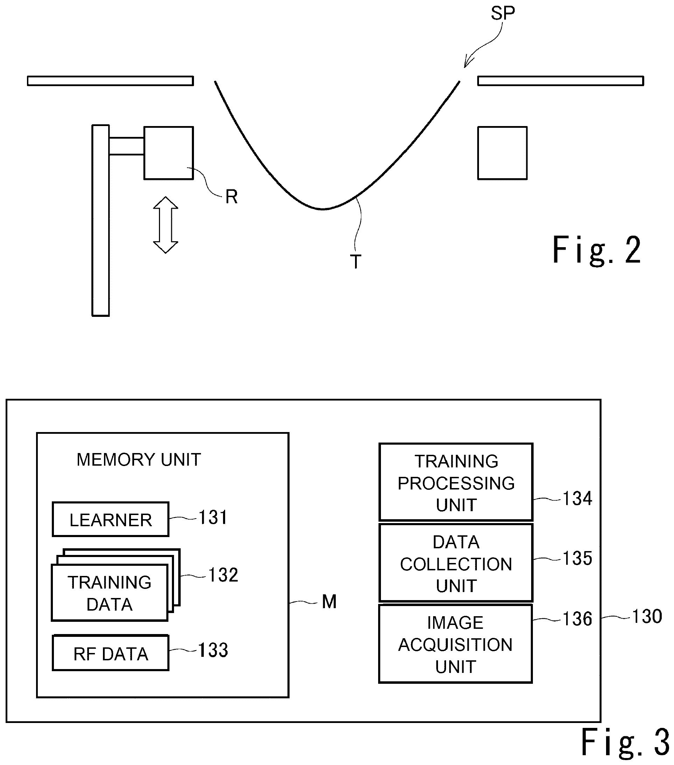

Fig. 2 is a section view taken along line A-A of Fig. 1. For example, the ring array R is installed under a bed having an opening such that the opening of the bed is superposed with an insertion portion SP. A test subject inserts a site of his or her body to be imaged (the test object T) into the insertion portion SP from the opening of the bed.

The insertion portion SP, into which the test object T is inserted, is provided at the center of the ring array R. The plurality of elements E of the ring array R are provided at equal intervals along the ring around the insertion portion SP. Convex lenses called acoustic lenses are attached to the inner peripheral side surface of the ring array R. Such surface treatment added on the inner peripheral side of the ring array R can cause ultrasound emitted by each element E to converge within a plane including the ring array R.

In the present embodiment, the elements E are arranged in a ring shape and at equal intervals; however, the shape of the ring array R is not limited to a circular shape and may be, for example, an arbitrary polygonal shape such as a hexagon, a square, and a triangle, a shape having at least partially a curve or an arc, another arbitrary shape, or a portion of these shapes (for example, a semicircle or an arc). That is, the ring array R can be generalized as an array R. In addition, the elements E constituting the array R are preferably arranged intermittently around the test object T so as to cover 90 degrees or more; however, the arrangement of the elements E is not limited to these.

The ring array R is connected to the emission-reception circuit 120 with the switch circuit 110 interposed therebetween. The emission-reception circuit 120 (control unit) transmits a control signal (electrical signal) to the elements E of the ring array R and controls emission and reception of ultrasound. For example, the emission-reception circuit 120 sends, to the elements E, a command as to for example the frequency and magnitude of ultrasound to be emitted and the type of wave (such as a continuous wave or a pulse wave).

The switch circuit 110 is connected to each of the plurality of elements E of the ring array R, transfers a signal from the emission-reception circuit 120 to certain elements E among the plurality of elements E, and drives the elements E to emit-receive a signal. For example, by switching the elements E to which the control signal from the emission-reception circuit 120 is supplied, the switch circuit 110 causes one of the plurality of elements E to function as an emission element, which emits ultrasound, and causes a plurality of elements E (for example, all the elements E) to receive reflected waves.

The ring array R is installed so as to be movable up and down by, for example, a stepping motor. Data on the entirety of the test object T can be collected by moving the ring array R up and down.

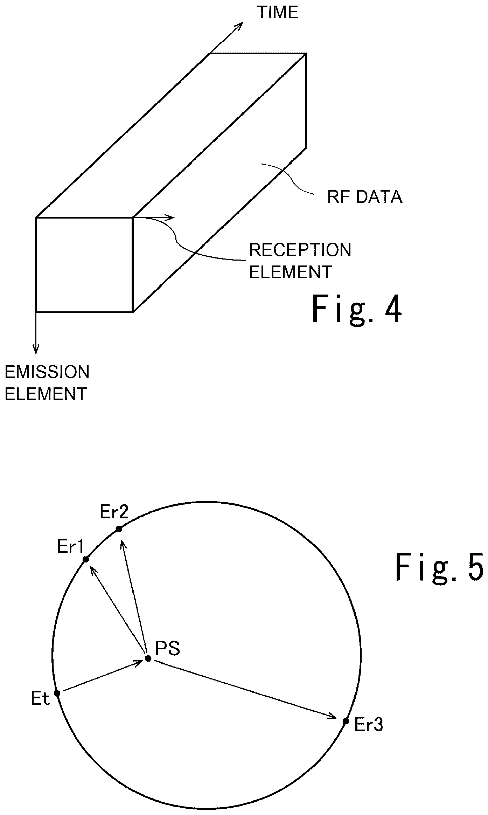

Next, measurement data (RF data), which is data obtained by a plurality of elements E, will be described. Ultrasound emitted from one emission element is reflected by the test object T and received by a plurality of reception elements. As a result, two-dimensional RF data is obtained in which a first axis represents reception-element number and a second axis represents reflected-wave propagation time. Pieces of two-dimensional data, the number of which is equal to the number of emission elements, is obtained by performing measurement while switching an emission element. In other words, three-dimensional RF data as illustrated in Fig. 4 is obtained in which a first axis represents reception-element number, a second axis represents reflected-wave propagation time, and a third axis represents emission-element number.

Fig. 5 is a schematic diagram illustrating the way in which ultrasound emitted from one emission element Et is reflected (scattered) by one point scatterer PS (one point on the test object T) and is received by a plurality of reception elements Er1, Er2, and Er3. Since ultrasound propagation paths (propagation distances) differ from each other, the reflected-wave propagation times to the respective reception elements differ from each other.

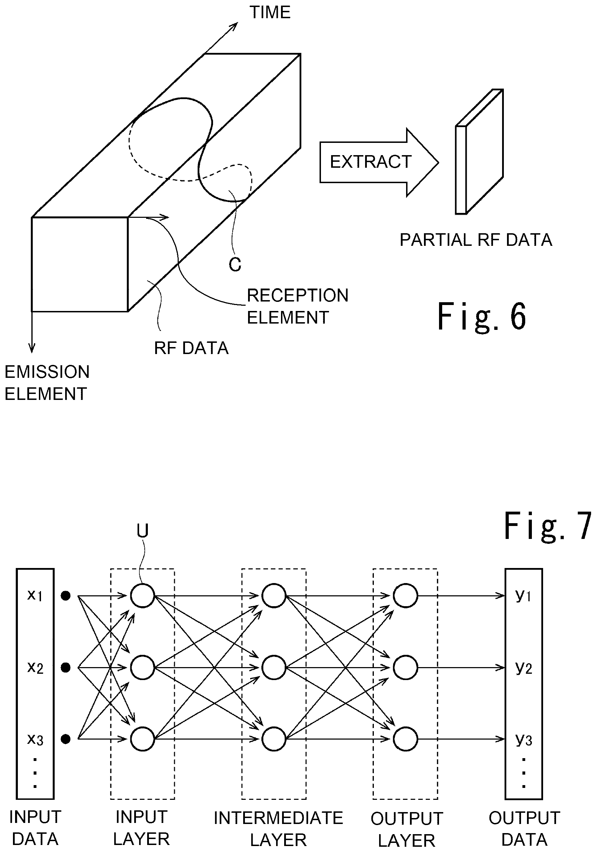

Thus, the waves reflected by the point scatterer PS and measured at the respective reception elements form a curve in two-dimensional RF data, in which the first axis represents reception-element number and the second axis represents reflected-wave propagation time. Moreover, the waves reflected by the point scatterer PS and measured at the respective reception elements are distributed on a curved surface C as illustrated in Fig. 6 in three-dimensional RF data. In the present embodiment, partial RF data corresponding to the curved surface C is extracted, the partial RF data is input to a trained learner, and a partially reconstructed image corresponding to the point scatterer PS is output.

As illustrated in Fig. 3, the calculation device 130 is constituted by, for example, a computer including a central processing unit (CPU), a communication unit, and a memory unit M. The memory unit M has, for example, a random-access memory (RAM), a read-only memory (ROM), and a hard disk. The functions of, for example, a data collection unit 135 and an image acquisition unit 136 are realized by executing an image reconstruction program stored in the memory unit M, and a measurement data storage area 133 is reserved in the memory unit M. Processing performed by each unit will be described later.

A learner 131 is stored in the memory unit M. The learner 131 is a processing execution program for performing processing on parameters such as weights and a bias regarding each unit (neuron) as well as input data. "A learner 131 is stored in the memory unit M" means that various parameters and the processing execution program regarding the learning 131 are stored in the memory unit M.

A training processing unit 134 executes training processing for the learner 131 using training data 132 stored in the memory unit M. The training data 132 includes RF data from a simulation regarding a biological model (test-object model) and a measurement image of the biological model (an ideal measurement image calculated from acoustic features of the biological model such as the strength of spatial gradient of acoustic impedance), the biological model being expressed by a distribution of acoustic features. In the simulation, the size and arrangement of the plurality of elements E of the ring array R are simulated in a simulation space and reflected ultrasound from the biological model is received by a plurality of elements while switching an emission element. A plurality of sets of simulation RF data and a measurement image are prepared.

The simulation RF data is input to the learner 131, and the learner 131 is trained such that output data from the learner 131 matches pixel values of the measurement image.

The learner 131 uses a neural network. Fig. 7 is a diagram illustrating the structure of the learner 131. Input data for the learner 131 includes a plurality of input variables $x_1$, $x_2$, $x_3$, .... The input variables of each input data are values of reception signals of the respective reception elements included in the simulation RF data.

The learner 131 is structured to include a plurality of layers each including a plurality of units U. Normally, the learner 131 is structured to include an input layer, an output layer, and an intermediate layer. The input layer is positioned on the side closest to the input. The output layer is positioned on the side closest to the output. The intermediate layer is provided between the input layer and the output layer. There is one intermediate layer in the example in Fig. 7; however, there may be a plurality of intermediate layers.

Each input variable is input to each unit U of the input layer. In each unit U, weights $w_1$, $w_2$, $w_3$, ... for the respective input variables and a bias b are defined. The value obtained by adding the bias to the sum of values each of which is obtained by multiplying a corresponding one of the input variables by its corresponding weight is an input u of the unit U.

Each unit 50 outputs an output f(u) of a function f called an activation function with respect to the input u. As the activation function, for example, a sigmoid function, a ramp function, a step function, or the like can be used. An output from each unit U of the input layer is input to each unit of the intermediate layer. That is, all of the units U of the input layer and all of the units of the intermediate layer are connected to each other.

Each unit of the intermediate layer receives, as an input, an output from each unit U of the input layer and performs substantially the same processing described above. That is, in each unit of the intermediate layer, weights corresponding to the respective units U of the input layer and a bias are set. An output from each unit of the intermediate layer is input to each unit of the output layer. That is, all of the units of the intermediate layer and all of the units of the output layer are connected to each other.

Each unit of the output layer receives, as an input, an output from each unit of the intermediate layer and performs substantially the same processing described above. That is, in each unit of the output layer, weights corresponding to the respective units of the intermediate layer and a bias are set.

An output from each unit of the output layer is output data from the learner 131. Output variables $y_1$, $y_2$, $y_3$, ... included in the output data become pixel values of pixels of a reconstructed image.

The training processing 134 adjusts the weights and bias of each unit of each layer such that the values of the output variables in the output data corresponding to the simulation RF data approach pixel values of a measurement image.

The data collection unit 135 collects (including receives or acquires) measurement data (RF data), which are data obtained by the plurality of elements, via the switch circuit 110 and the emission-reception circuit 120. The RF data is stored in the RF data storage area 133 of the memory unit M.

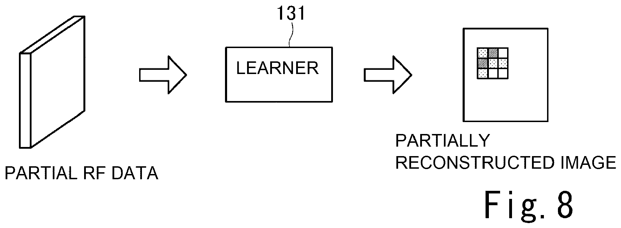

The image acquisition unit 136 extracts, from the RF data, partial RF data corresponding to a point of interest. As illustrated in Fig. 8, the image acquisition unit 136 inputs, as input data, partial RF data to the learner 131, which has been sufficiently trained, and acquires pixel values of a partially reconstructed image output from the learner 131. The image acquisition unit 136 repeatedly performs the processing described above for a plurality of points of interest included in a region of interest (ROI). As a result, a tomographic image corresponding to the ROI of the test object T can be reconstructed. The reconstructed image is displayed on the image display device 140. In particular, in the present invention, a data generation time for learning can be greatly improved by setting an enormous number of points of interest on one image. For example, in a case where the number of pixels is $N^2$ and the distance between adjacent pixels for which the independence of information for each pixel is not guaranteed is n, $N^2/n^2$ points of interest can be set as an order and the temporal cost and data size required for the numerical simulation can be significantly reduced.

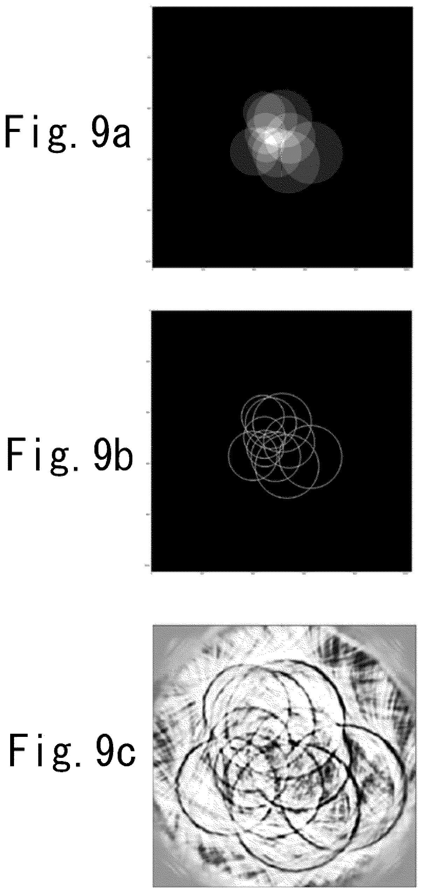

Fig. 9a illustrates an example of a biological model, which is a material density distribution. Fig. 9b illustrates a measurement image of this biological model. Fig. 9c illustrates an example of a reconstructed image obtained from output data by inputting simulation RF data for this biological model to the learner 131, which has already been trained. It was confirmed that it was possible to obtain a clear reconstructed image.

In this manner, according to the present embodiment, since RF data (partial RF data) is simply input to the learner 131 without performing an approximation calculation or the like thereon, accurate image reconstruction can be performed without reducing the amount of information of the RF data. Moreover, according to the present embodiment, training data generated through a simulation can be used to train the learner 131. Here, in a case where a clinical image is used as training data, a huge number of clinical images with doctors' diagnosis results are generally required in order to ensure the accuracy of learning. However, it is not easy to collect a necessary and sufficient number of clinical images. Moreover, as a result of using only data for which diagnoses are made by human beings, images outside the range of existing image data cannot be learned, and thus it is difficult to completely eliminate sample bias of data to be used. Therefore, use of training data generated through a simulation can suppress the generation cost of training data and reduce the training-data bias and variation in accuracy.

The memory unit M may further store a second learner. The second learner learns using training data including tomographic images (for example, past medical images) and tumor information determining the positions of tumors included in the tomographic images and can determine the presence or absence and position of a tumor in tomographic images of a test object.

The calculation device 130 further includes a determination unit. The determination unit inputs a tomographic image acquired by the image acquisition unit 136 to the second learner and outputs, on the basis of output data from the second learner, a determination result on the presence or absence of a tumor in the tomographic image and, in a case where a tumor exists, information on the position of the tumor.

Partial RF data to be input to the learner 131 may include not only RF data corresponding to the curved surface C as illustrated in Fig. 6 but also RF data of an area surrounding the curved surface C.

When the number of emission conditions N, the number of reception elements M, and the number of sample points n in the time direction are used, the size of partial RF data illustrated in Fig. 6 is N × M × n. In the description made so far, which value to which n is set has not been described in detail. Under conditions where the effect caused by the heterogeneity of sound speed is small, n = 1 or n can be set to a value close to 1. In contrast, under conditions where the effect caused by the heterogeneity of sound speed is large, uncertainty increases when the distances illustrated in Fig. 5 are converted into propagation times. Thus, it is preferable that n be set to a relatively large value. For example, a value is obtained by dividing the difference between a propagation time when the sound speed is fastest and a propagation time when the sound speed is slowest in a medium that may be present in the paths by a sampling period, and n is set to a value obtained by multiplying the value by the dispersion in propagation time. As an effect obtained through learning using the structure according to the present invention, the heterogeneity of sound speed can be expected to be corrected by setting n in this manner.

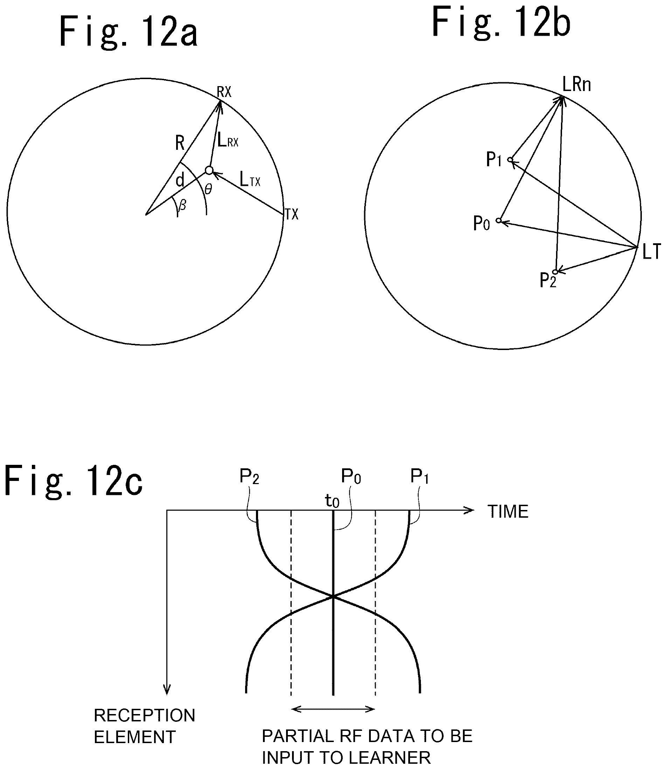

Fig. 12a is a diagram illustrating an example of ultrasound propagation paths. When reflected waves that are waves emitted from an emission point LT and reflected by each scatter point PI are received at a reception point LR, a propagation time from the emission point LT to the reception point LR can be obtained by the following Equation 1, in which the radius of the area of a circle surrounded by the ring array is R, the distance between the center of the circle and the scatter point PI is d, the distance from the emission point LT to the scatter point PI is $L_{TX}$, the distance from the scatter point PI to the reception point LR is $L_{RX}$, the position of the emission point LT is $(R, \omega)$, and the position of the reception point is $(R, \theta)$.

[Math 1]

$$L_{TX} = \sqrt{R^2 + d^2 - 2Rd \cos[\omega - \beta]}$$
$$L_{RX} = \sqrt{R^2 + d^2 - 2Rd \cos[\theta - \beta]} \quad \cdots (1)$$

Since the propagation time is proportional to distance, a propagation time at a certain time t can be expressed as in the following Equation 2.

[Math 2]

$$t \propto L_{TwoWay} = L_{TX} + L_{RX} \quad \cdots (2)$$

Fig. 12b is a diagram illustrating an example of ultrasound propagation paths in a case where reflected waves that are waves emitted from the emission point LT and reflected by point scatterers $P_0$, $P_1$, and $P_2$ are received at a reception point LRn. Fig. 12c illustrates RF data in a case where the emission point LT is used. The vertical axis represents reception-element number, and the horizontal axis is a time axis (reflected-wave propagation time).

For example, in a case where the position of the point scatterer $P_0$ is to be determined, RF data at a time $t_0$ includes not only a reflected-wave component from the point scatterer $P_0$ but also reflected-wave components from the point scatterers $P_1$ and $P_2$. Thus, preferably, partial RF data to be input to the learner 131 is not data only at the time $t_0$ but is data having a width to some extent and including the time $t_0$ (the number of sample points n in the time direction is increased). The RF data that each scatter point has includes information unique to the scatter point. For example, the RF data has a unique trajectory based on the position of the scatter point and has a signal strength that varies depending on the scattering intensity dependent on the characteristics of living tissue that the scatter point has. Thus, by using information that is continuous over time, it becomes easier to detect effects due to the reflected-wave components from characteristics of the RF data of each of the point scatters $P_1$ and $P_2$. Consequently, the image quality of a partially reconstructed image corresponding to the point scatterer $P_0$, which is a point scatterer of interest, can be improved. It is difficult to distinguish partial RF data to be input to the learner 131 from other RF data if the temporal width of the partial RF data is too small, and the man-hour cost for deep learning increases if the temporal width is too large. Therefore, preferably, a width is selected that sufficiently satisfies both conditions.

Next, an embodiment will be described in which the present invention is used for another application other than correction of the heterogeneity of sound speed. In imaging using a ring array, when the number of emission conditions is increased, the imaging time increases and the size of acquired RF data increases. In RF data for which an appropriate sampling frequency is set with respect to the center frequency, in a case where the number of emission conditions is on the order of 100 and the number of reception elements is on the order of 1000, the data size per cross section is on the order of a few hundred GB. The amount of three-dimensional data for an entire breast ends up being a significantly massive amount of data as large as 1 TB.

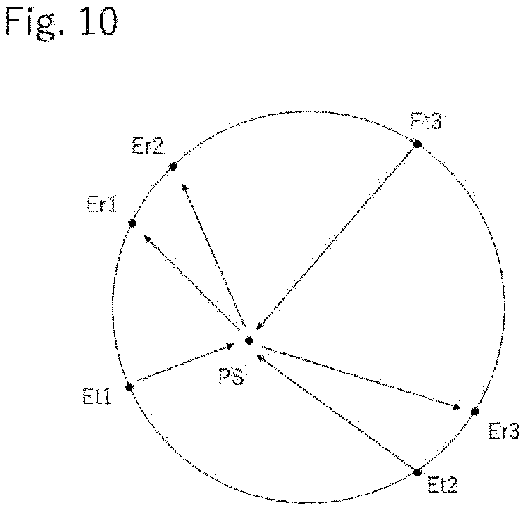

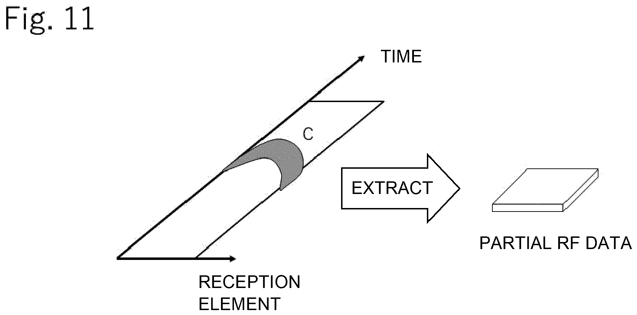

In contrast, in order not to create a region where the signal-to-noise ratio decreases, it is effective to introduce sound energy into the entire imaging region through provision of ultrasonic energy from multiple directions. In an existing synthetic aperture method, a propagation time is obtained by determining which emission point (Et), which scatter point (PS), and which reception point (Er) are to be used, so that imaging can be performed. However, according to a method of the present invention, when emission is performed simultaneously from a plurality of emission points Et (the drawing illustrates an example of three emission points Et) and reception is performed at each Er as illustrated in Fig. 10, a strip-shaped region is a region where corresponding data is stored as illustrated in Fig. 11. (As a matter of course, the data in this strip is not constituted only by the corresponding data).

The line C is spread out into a strip-shaped region in an existing synthetic aperture method, thereby resulting in a blurred image. In contrast, in the present invention, by extracting partial RF data from this strip-shaped data and learning the partial RF data, it becomes possible to perform imaging while suppressing blurring and suppressing inconsistencies in the signal-to-noise ratio inside the region due to the small number of emission conditions. As a result of this, the imaging time can be reduced and the total data amount of RF data can be reduced.

Note that, in the present invention, a simulation calculation for ultrasound propagating through a biological model can be performed by solving a wave equation using the finite-difference time-domain method or by performing a calculation using a method such as ray tracing. The biological model is obtained by discretizing a space and setting, for example, a sound speed, a density, and an attenuation factor at each discrete point. Here, scattering of ultrasound is given by the spatial gradient of acoustic impedance, which is the product of the sound speed and the density, and thus the attenuation factor is given as disturbance similarly to the heterogeneity of sound speed. To perform robust learning for such disturbance is also an advantage of learning using various biological models.

To train the learner 131, a brightness image, which is a gray scale image into which a natural image such as an image of an animal or a landscape is converted, may be used as training data instead of a biological model using a simulation. This is because a natural image includes various spatial frequency components and thus provides an environment more similar to that in a living body that is an imaging target in a medical image. When random pattern images are artificially generated to construct a biological model for training, higher spatial frequency components are relatively more likely to be present than lower spatial frequency components, so that there are differences between features of the biological model and features that should be learned in training as clinical images. If a large number of clinical images are available, this is appropriate for the objective; however, it is generally difficult to collect a large number of clinical images, and clinical images do not have sufficiently high spatial frequencies because the spatial resolution of clinical images is limited. Therefore, clinical images are not always appropriate as a training model. Use of natural images is advantageous in terms of the two points described above.

In this case, first, a brightness image is converted into an acoustic feature distribution image. Note that, in the present embodiment, an example will be described in which a density distribution image is used as an acoustic feature distribution image; however, for example, a sound speed distribution image or the like can also be applied. The following equation can be used to perform image conversion. In the following equation, $\sigma_i(x, y)$ is a density corresponding to a pixel $(x, y)$, $\sigma_0$ is a reference density, $I(x, y)$ is a brightness (pixel value) of $[0, 1]$ at the pixel $(x, y)$ of the brightness image, $\sigma_{max}$ is a maximum amplitude, and $\varepsilon$ is a random number of $[-1, 1]$.

$$\sigma_i(x, y) = \sigma_0 + I(x, y) * \sigma_{max} * \varepsilon$$

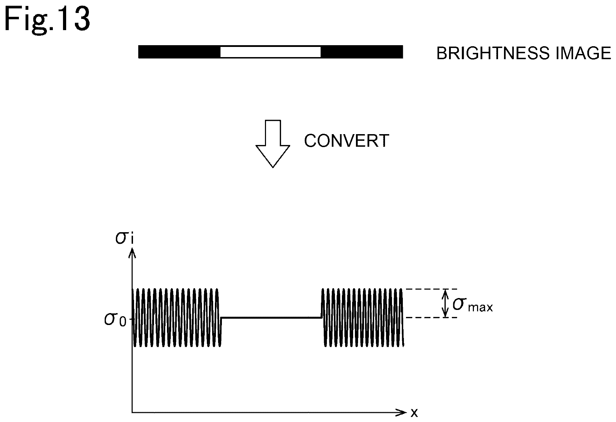

Fig. 13 illustrates an example in which a pixel sequence (a brightness image) is converted into a density distribution. In the pixel sequence, a white portion, a black portion, and a white portion are arranged in order.

In a simulation space, the size and arrangement of the plurality of elements E of the ring array R are simulated for the density distribution, and RF data (measurement data) of a simulation is input to the learner 131. In the simulation, reflected ultrasound from the density distribution is received by a plurality of elements while switching an emission element. The learner 131 is trained such that output data from the learner 131 match pixel values of the brightness image.

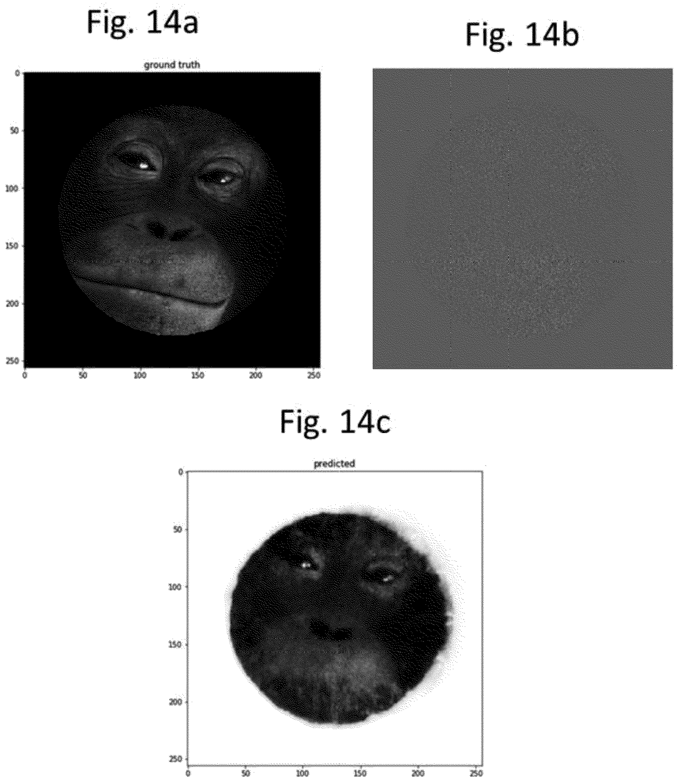

Fig. 14a illustrates a brightness image that is a gray scale image into which an image of a chimpanzee is converted. Fig. 14b illustrates a density distribution image generated from the brightness image of Fig. 14a. Fig. 14c illustrates a reconstructed image obtained from output data from the learner 131, which has already been trained and into which simulation RF data for the density distribution of Fig. 14b is input. It was confirmed that it was possible to obtain a clear reconstructed image.

In the embodiment described above, the configuration using a ring array has been described; however, a probe may also be used on which elements that emit and receive ultrasound are arranged in a straight line or a plane.

The present invention has been described in details using specific embodiments; however, it is obvious to those skilled in the art that various changes can be made within the scope of the appended claims.

This application is based on Japanese Patent Application No. 2018-198658 filed October 22, 2018.

Reference Signs List

10      ultrasound diagnostic system
110     switch circuit
120     emission-reception circuit
130     calculation device
140     image display device

**Claims**

1.  An ultrasound computed tomography diagnostic system (10) comprising:

    a plurality of elements (E) configured to perform at least one of emission of ultrasound to a test object (T) and reception of reflected ultrasound that is ultrasound reflected from the test object (T);
    a data collection unit (135) configured to collect measurement data of the reflected ultrasound through at least one of the plurality of elements (E) while switching an element that emits ultrasound;
    an image acquisition unit (136) configured to perform steps of extracting, from the collected measurement data, partial measurement data corresponding to a point of interest, inputting the partial measurement data to a first learner (131), and acquiring pixel values of a partially reconstructed image output from the first learner (131), wherein the image acquisition unit (136) is configured to repeatedly perform the steps for a plurality of points of interest included in a region of interest in order to reconstruct a tomographic image corresponding to the region of interest of the test object (T);
    an image display device (140) configured to display said reconstructed tomographic image corresponding to the region of interest of the test object (T); and
    the first learner (131) using a neural network, the first learner (131) being configured to learn using training data (132) including a test-object model and simulation measurement data and to output the partially reconstructed image from the partial measurement data which is input to the first learner (131), the test-object model being expressed by a distribution of acoustic features, the simulation measurement data being obtained by emitting ultrasound while switching an element that emits ultrasound and receiving reflected ultrasound from the test-object model using at least one of the plurality of elements (E) in a simulation space in which a size and an arrangement of the plurality of elements (E) are simulated.

2.  An ultrasound computed tomography diagnostic system (10) comprising:

    a plurality of elements (E) configured to perform at least one of emission of ultrasound to a test object (T) and reception of reflected ultrasound that is ultrasound reflected from the test object (T);
    a data collection unit (135) configured to collect measurement data of the reflected ultrasound through at least one of the plurality of elements (E) while switching an element that emits ultrasound;
    an image acquisition unit (136) configured to perform steps of extracting, from the collected measurement data, partial measurement data corresponding to a point of interest, inputting the partial measurement data to a first learner (131), and acquiring pixel values of a partially reconstructed image output from the first learner (131), wherein the image acquisition unit (136) is configured to repeatedly perform the steps for a plurality of points of interest included in a region of interest in order to reconstruct a tomographic image corresponding to the region of interest of the test object (T);
    an image display device (140) configured to display said reconstructed tomographic image corresponding to the region of interest of the test object (T); and
    the first learner (131) using a neural network, the first learner (131) being configured to learn using training data (132) including a brightness image and simulation measurement data and to output the partially reconstructed image from the partial measurement data which is input to the first learner (131), the brightness image being a gray scale image into which a natural image is converted, the simulation measurement data being obtained by emitting ultrasound while switching an element that emits ultrasound and receiving reflected ultrasound from an acoustic feature distribution based on the brightness image using at least one of the plurality of elements (E) in a simulation space in which a size and an arrangement of the plurality of elements (E) are simulated.

3. The ultrasound computed tomography diagnostic system (10) according to Claim 1 or 2, further **characterized by** comprising:

a second learner capable of determining a presence or absence of and a position of a tumor in a tomographic image of a test object (T) through learning using training data including a training tomographic image and tumor information determining a position of a tumor included in the training tomographic image; and
a determination unit configured to input the tomographic image acquired by the image acquisition unit (136) to the second learner and outputs, on a basis of output data from the second learner, a determination result on a presence or absence of a tumor in the tomographic image and, in a case where a tumor exists, information on a position of the tumor.

4. The ultrasound computed tomography diagnostic system (10) according to any one of Claims 1 to 3, **characterized in that** the plurality of elements (E) are arranged around the test object (T).

**Patentansprüche**

1. Ultraschall-Computertomographie-Diagnosesystem (10), umfassend:

eine Vielzahl von Elementen (E), die so ausgebildet sind, dass sie mindestens eines von Aussenden von Ultraschall zu einem Testobjekt (T) und Empfangen von reflektiertem Ultraschall, der vom Testobjekt (T) reflektierter Ultraschall ist, durchführen;
eine Datensammeleinheit (135), die so ausgebildet ist, dass sie Messdaten des reflektierten Ultraschalls durch mindestens eines der Vielzahl von Elementen (E) sammelt, während sie ein Element, das Ultraschall aussendet, umschaltet;
eine Bilderfassungseinheit (136), die so ausgebildet ist, dass sie die Schritte des Extrahierens von Teilmessdaten, die einem interessierenden Punkt entsprechen, aus den gesammelten Messdaten, des Eingebens der Teilmessdaten in einen ersten Lerner (131) und des Erfassens von Pixelwerten eines teilweise rekonstruierten Bildes, das von dem ersten Lerner (131) ausgegeben wird, durchführt, wobei die Bilderfassungseinheit (136) so ausgebildet ist, dass sie die Schritte für eine Vielzahl von interessierenden Punkten, die in einem interessierenden Bereich eingeschlossen sind, wiederholt durchführt, um ein tomographisches Bild zu rekonstruieren, das dem interessierenden Bereich des Testobjekts (T) entspricht;
eine Bildanzeigevorrichtung (140), die so ausgebildet ist, dass sie das rekonstruierte tomographische Bild, das dem interessierenden Bereich des Testobjekts (T) entspricht, anzeigt; und
wobei der erste Lerner (131) ein neuronales Netzwerk verwendet, wobei der erste Lerner (131) so ausgebildet ist, dass er unter Verwendung von Trainingsdaten (132), die ein Testobjektmodell und Simulationsmessdaten einschließen, lernt und dass er das teilweise rekonstruierte Bild aus den Teilmessdaten ausgibt, die in den ersten Lerner (131) eingegeben werden, wobei das Testobjektmodell durch eine Verteilung akustischer Merkmale ausgedrückt wird, wobei die Simulationsmessdaten durch Aussenden von Ultraschall während des Umschaltens eines Elements, das Ultraschall aussendet, und Empfangen von reflektiertem Ultraschall von dem Testobjektmodell unter Verwendung mindestens eines der Vielzahl von Elementen (E) in einem Simulationsraum, in dem eine Größe und eine Anordnung der Vielzahl von Elementen (E) simuliert werden, empfangen werden.

2. Ultraschall-Computertomographie-Diagnosesystem (10), umfassend:

eine Vielzahl von Elementen (E), die so ausgebildet sind, dass sie zumindest eines von Aussenden von Ultraschall zu einem Testobjekt (T) und Empfangen von reflektiertem Ultraschall, der vom Testobjekt (T) reflektierter Ultraschall ist, durchführen;
eine Datensammeleinheit (135), die so ausgebildet ist, dass sie Messdaten des reflektierten Ultraschalls durch mindestens eines der Vielzahl von Elementen (E) sammelt, während sie ein Element, das Ultraschall aussendet, umschaltet;
eine Bilderfassungseinheit (136), die so ausgebildet ist, dass sie die Schritte des Extrahierens von Teilmessdaten, die einem interessierenden Punkt entsprechen, aus den gesammelten Messdaten, des Eingebens der Teilmessdaten in einen ersten Lerner (131) und des Erfassens von Pixelwerten eines teilweise rekonstruierten Bildes, das von dem ersten Lerner (131) ausgegeben wird, durchführt, wobei die Bilderfassungseinheit (136) so ausgebildet ist, dass sie die Schritte für eine Vielzahl von interessierenden Punkten, die in einem interessierenden Bereich eingeschlossen sind, wiederholt durchführt, um ein tomographisches Bild zu rekonstruieren, das dem interessierenden Bereich des Testobjekts (T) entspricht;

eine Bildanzeigevorrichtung (140), die so ausgebildet ist, dass sie das rekonstruierte tomographische Bild, das dem interessierenden Bereich des Testobjekts (T) entspricht, anzeigt; und

der erste Lerner (131) ein neuronales Netzwerk verwendet, wobei der erste Lerner (131) so ausgebildet ist, dass er unter Verwendung von Trainingsdaten (132), die ein Helligkeitsbild und Simulationsmessdaten einschließen, lernt und dass er das teilweise rekonstruierte Bild aus den Teilmessdaten ausgibt, die in den ersten Lerner (131) eingegeben werden, wobei das Helligkeitsbild ein Graustufenbild ist, in das ein natürliches Bild umgewandelt wird, die Simulationsmessdaten durch Aussenden von Ultraschall während des Umschaltens eines Elements, das Ultraschall aussendet, und Empfangen von reflektiertem Ultraschall von einer akustischen Merkmalsverteilung auf der Grundlage des Helligkeitsbildes unter Verwendung mindestens eines der Vielzahl von Elementen (E) in einem Simulationsraum, in dem eine Größe und eine Anordnung der Vielzahl von Elementen (E) simuliert werden, empfangen werden.

3. Ultraschall-Computertomographie-Diagnosesystem (10) nach Anspruch 1 oder 2, weiter **dadurch gekennzeichnet, dass** es Folgendes umfasst:

einen zweiten Lerner, der in der Lage ist, ein Vorhandensein oder Nichtvorhandensein und eine Position eines Tumors in einem tomographischen Bild eines Testobjekts (T) durch Lernen unter Verwendung von Trainingsdaten zu bestimmen, die ein tomographisches Trainingsbild und Tumorinformationen einschließen, die eine Position eines Tumors bestimmen, der in dem tomographischen Trainingsbild eingeschlossen ist; und

eine Bestimmungseinheit, die so ausgebildet ist, dass sie das von der Bilderfassungseinheit (136) erfasste tomographische Bild in den zweiten Lerner eingibt und auf der Grundlage von Ausgabedaten von dem zweiten Lerner ein Bestimmungsergebnis über ein Vorhandensein oder Nichtvorhandensein eines Tumors in dem tomographischen Bild und, in einem Fall, in dem ein Tumor vorhanden ist, Informationen über eine Position des Tumors ausgibt.

4. Ultraschall-Computertomographie-Diagnosesystem (10) nach einem der Ansprüche 1 bis 3, **dadurch gekennzeichnet, dass** die Vielzahl von Elementen (E) um das Testobjekt (T) herum angeordnet sind.

**Revendications**

1. Système (10) de diagnostic de tomodensitométrie à ultrasons comprenant :

une pluralité d'éléments (E) configurés pour mettre en oeuvre au moins une parmi une émission d'ultrasons vers un objet test (T) et une réception d'ultrasons réfléchis qui sont des ultrasons réfléchis depuis l'objet test (T) ;

une unité de collecte (135) de données configurée pour collecter des données de mesure des ultrasons réfléchis par le biais d'au moins un de la pluralité d'éléments (E) pendant une commutation d'un élément qui émet des ultrasons ;

une unité d'acquisition (136) d'image configurée pour mettre en oeuvre des étapes d'extraction, à partir des données de mesure collectées, de données de mesure partielle correspondant à un point d'intérêt, d'entrée des données de mesure partielle dans un premier dispositif d'apprentissage (131), et d'acquisition de valeurs de pixel d'une image partiellement reconstruite fournie en sortie du premier dispositif d'apprentissage (131), dans lequel l'unité d'acquisition (136) d'image est configurée pour mettre en oeuvre de façon répétée les étapes pour une pluralité de points d'intérêt inclus dans une région d'intérêt afin de reconstruire une image tomographique correspondant à la région d'intérêt de l'objet test (T) ;

un dispositif d'affichage (140) d'image configuré pour afficher ladite image tomographique reconstruite correspondant à la région d'intérêt de l'objet test (T) ; et

le premier dispositif d'apprentissage (131) utilisant un réseau neuronal, le premier dispositif d'apprentissage (131) étant configuré pour apprendre à l'aide de données d'entraînement (132) incluant un modèle d'objet test et de données de mesure de simulation et pour fournir en sortie l'image partiellement reconstruite à partir des données de mesure partielle qui sont entrées dans le premier dispositif d'apprentissage (131), le modèle d'objet test étant exprimé par une distribution de caractéristiques acoustiques, les données de mesure de simulation étant obtenues en émettant des ultrasons pendant une commutation d'un élément qui émet des ultrasons et en recevant des ultrasons réfléchis depuis le modèle d'objet test à l'aide d'au moins un de la pluralité d'éléments (E) dans un espace de simulation dans lequel une taille et un agencement de la pluralité d'éléments (E) sont simulés.

2. Système (10) de diagnostic de tomodensitométrie à ultrasons comprenant :

une pluralité d'éléments (E) configurés pour mettre en oeuvre au moins une parmi une émission d'ultrasons vers un objet test (T) et une réception d'ultrasons réfléchis qui sont des ultrasons réfléchis depuis l'objet test (T) ;
une unité de collecte (135) de données configurée pour collecter des données de mesure des ultrasons réfléchis par le biais d'au moins un de la pluralité d'éléments (E) pendant une commutation d'un élément qui émet des ultrasons ;
une unité d'acquisition (136) d'image configurée pour mettre en oeuvre des étapes d'extraction, à partir des données de mesure collectées, de données de mesure partielle correspondant à un point d'intérêt, d'entrée des données de mesure partielle dans un premier dispositif d'apprentissage (131), et d'acquisition de valeurs de pixel d'une image partiellement reconstruite fournie en sortie du premier dispositif d'apprentissage (131), dans lequel l'unité d'acquisition (136) d'image est configurée pour mettre en oeuvre de façon répétée les étapes pour une pluralité de points d'intérêt inclus dans une région d'intérêt afin de reconstruire une image tomographique correspondant à la région d'intérêt de l'objet test (T) ;
un dispositif d'affichage (140) d'image configuré pour afficher ladite image tomographique reconstruite correspondant à la région d'intérêt de l'objet test (T) ; et
le premier dispositif d'apprentissage (131) utilisant un réseau neuronal, le premier dispositif d'apprentissage (131) étant configuré pour apprendre à l'aide de données d'entraînement (132) incluant une image de luminosité et de données de mesure de simulation et pour fournir en sortie l'image partiellement reconstruite à partir des données de mesure partielle qui sont entrées dans le premier dispositif d'apprentissage (131), l'image de luminosité étant une image à niveaux de gris dans laquelle une image naturelle est convertie, les données de mesure de simulation étant obtenues en émettant des ultrasons pendant une commutation d'un élément qui émet des ultrasons et en recevant des ultrasons réfléchis depuis une distribution de caractéristiques acoustiques sur la base de l'image de luminosité à l'aide d'au moins un de la pluralité d'éléments (E) dans un espace de simulation dans lequel une taille et un agencement de la pluralité d'éléments (E) sont simulés.

3. Système (10) de diagnostic de tomodensitométrie à ultrasons selon la revendication 1 ou la revendication 2, **caractérisé en outre en ce qu'**il comprend :

un second dispositif d'apprentissage apte à déterminer une présence ou absence et une position d'une tumeur dans une image tomographique d'un objet test (T) à travers un apprentissage à l'aide de données d'entraînement incluant une image tomographique d'entraînement et d'informations de tumeur déterminant une position d'une tumeur incluse dans l'image tomographique d'entraînement ; et
une unité de détermination configurée pour entrer l'image tomographique acquise par l'unité d'acquisition (136) d'image dans le second dispositif d'apprentissage et fournit en sortie, sur la base de données de sortie provenant du second dispositif d'apprentissage, un résultat de détermination sur une présence ou absence d'une tumeur dans l'image tomographique et, dans un cas où une tumeur existe, des informations sur une position de la tumeur.

4. Système (10) de diagnostic de tomodensitométrie à ultrasons selon l'une quelconque des revendications 1 à 3, **caractérisé en ce que** la pluralité d'éléments (E) sont agencés autour de l'objet test (T).

Fig. 1

SP

R

T

Fig. 2

| MEMORY UNIT | | | |
|---|---|---|---|
| LEARNER | ~131 | TRAINING PROCESSING UNIT | ~134 |
| TRAINING DATA | ~132 | DATA COLLECTION UNIT | ~135 |
| RF DATA | ~133 | IMAGE ACQUISITION UNIT | ~136 |

MEMORY UNIT

LEARNER ~131

TRAINING DATA ~132

RF DATA ~133

M

TRAINING PROCESSING UNIT ~134

DATA COLLECTION UNIT ~135

IMAGE ACQUISITION UNIT ~136

~130

Fig. 3

TIME

RF DATA

RECEPTION
ELEMENT

Fig. 4

EMISSION
ELEMENT

Er2

Er1

Fig. 5

PS

Et

Er3

TIME

EXTRACT

PARTIAL RF DATA

C

RECEPTION
ELEMENT

**Fig. 6**

RF DATA

EMISSION
ELEMENT

**Fig. 7**

U

$x_1$

$x_2$

$x_3$

$y_1$

$y_2$

$y_3$

INPUT
DATA

INPUT
LAYER

INTERMEDIATE
LAYER

OUTPUT
LAYER

OUTPUT
DATA

131

LEARNER

PARTIAL RF DATA

PARTIALLY
RECONSTRUCTED IMAGE

Fig. 8

Fig. 9a

Fig. 9b

Fig. 9c

Fig. 10

Fig. 11

TIME

C

EXTRACT

PARTIAL RF DATA

RECEPTION
ELEMENT

# Fig. 12a

# Fig. 12b

# Fig. 12c

RECEPTION
ELEMENT

PARTIAL RF DATA TO BE
INPUT TO LEARNER

TIME

# Fig.13

BRIGHTNESS IMAGE

CONVERT

$\sigma i$

$\sigma_0$

$\sigma_{max}$

x

## Fig. 14a

ground truth

## Fig. 14b

## Fig. 14c

predicted

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2017051903 A
- JP 2018198658 A

**Non-patent literature cited in the description**

- A Deep Learning Framework for Single-Sided Sound Speed Inversion in Medical Ultrasound. **MICHA FEIGIN et al.** ARXIV.ORG. CORNELL UNIVERSITY LIBRARY, 30 September 2018
- **VEDULA, SANKETH et al.** TOWARDS CT-QUALITY ULTRASOUND IMAGING USING DEEP LEARNING. *arXiv, 1710.06304v1,* 17 October 2017
- **CHENG, H. D et al.** Automated breast cancer detection and classification using ultrasound images= A survey. *Pattern Recognition,* 2010, vol. 43, 299-317